Europäisches Patentamt
European Patent Office
Office européen des brevets

Publication number: 0 287 000 A1

# EUROPEAN PATENT APPLICATION

Application number: 88105681.6

Int. Cl.⁴ A61K 35/78 , A61K 31/045

Date of filing: 09.04.88

Priority: 17.04.87 IT 2018187

Date of publication of application: 19.10.88 Bulletin 88/42

Designated Contracting States: ES GR

Applicant: CONSIGLIO NAZIONALE DELLE RICERCHE
Piazzale Aldo Moro, 7
I-00185 Roma(IT)

Applicant: INVERNI DELLA BEFFA S.P.A.
Via Ripamonti, 99
I-20141 Milano(IT)

Inventor: Jommi, Giancarlo
Dip.di Chimica Organica ed Industriale
Via Venezian 21 I-20100 Milano(IT)
Inventor: Verotta, Luisella
Dip.di Chimica Organica ed Industriale
Via Venezian 21 I-20100 Milano(IT)
Inventor: Magistretti, Maria José
Dip.di Chimica Organica ed Industriale
Via Venezian 21 I-20100 Milano(IT)

Representative: Bianchetti, Giuseppe
Studio Consulenza Brevettuale Via Rossini, 8
I-20122 Milan(IT)

## Pharmaceutical compositions containing higher alcohols for the treatment of prostatic pathologies.

Pharmaceutical compositions containing as the active principle the alcoholic fraction obtained from the total lipophilic extract of Serenoa repens fruits, after alkaline hydrolysis and extraction with water immiscible solvents.

The extract is endowed with a better antiandrogenic activity in comparison to the total lipophilic extract.

EP 0 287 000 A1

# PHARMACEUTICAL COMPOSITIONS CONTAINING HIGHER ALCOHOLS FOR THE TREATMENT OF PROSTATIC PATHOLOGIES

The invention refers to pharmaceutical compositions useful for treating pathologic conditions of the prostata containing a mixture of long chain alcohols, saturated and unsaturated, sterolic and triterpenic, extracted from Serenoa repens fruits, and to the respective processes of preparation.

The fruits of Serenoa repens (Bart.) Small, small palm also known as Sabal serrulatum and Sabal serrulata Roem. and Schult., typical of the south-eastern United States flora, have been used for a long time in phytotherapy for the treatment of functional troubles connected to prostatic hypertrophy. Their use is cited for instance in "Dispensatory of the United States of America" (J.B. Lippincot Co., Philadelphia, 1960, 25° Ed., 2, 1840) and is reported in the American Pharmacopeia (National Formulary VIII, 8° Ed., 457).

Processes of preparation of extracts of said fruits have been recently patented, disclosing the use of solvents having low polarity (FR-A-2.480.754) or of inert gas in hypercritical conditions (Italian patent application N. 20813 A 86).

By said processes, a total lipophilic extract, with a ponderal yield of 10% starting from the vegetal material, endowed with curative properties of illnesses caused by benign prostatic hypertrophy is obtained.

This extract is composed for 85% by a fatty acids mixture (essentially capronic, caprinic, decanoic, lauric, myristic, palmitic, oleic and stearic acids) and for 15% by a mixture of ethyl and methyl esters of said acids, by long-chain saturated and unsaturated alcohols in free and esterified form with the above acids and by sterols and triterpenic alcohols which are also present in free and esterified form. Particularly, the alcoholic fraction turns out to be composed by the alcohols n-docosanol ($C_{22}H_{45}OH$), n-tricosanol ($C_{23}H_{47}OH$), n-tetracosanol ($C_{24}H_{49}OH$), n-hexacosanol ($C_{26}H_{53}OH$), n-octacosanol ($C_{28}H_{57}OH$), phytal, farnesal, geranylgeraniol, stigmasterol, β-sitosterol, cycloartenol, lupeal, 24-methylenecycloartanol and four polyprenols having general structure (I), whose relative ratios will be reported hereinafter.

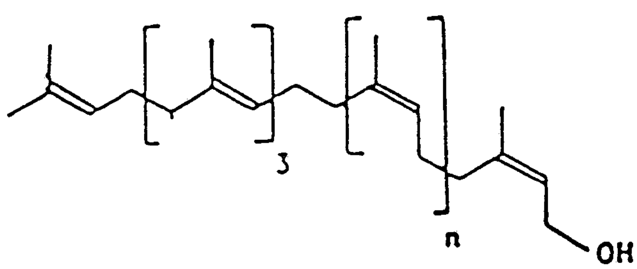

(I) n = 1-4

It has now been found that it is possible to enhance the pharmacological activity of the total extract by subjecting it to alkaline hydrolysis in suitable conditions and separating the alcoholic fraction from the acidic one, which is discarded.

According to a first embodiment of the process of the invention, the total lipophilic extract obtained according to known methods is treated with alkali in hydroalcoholic mixture and the acidic mixture is discarded by filtration of the formed salts. The mixture of the alcohols is obtained by extraction of the filtrate with a water immiscible solvent and evaporation to dryness of the organic phase.

Alternatively, when a decrease of the solvent consumption is desired, the total lipophilic extract may be preventively purified from the acidic component by dissolution of the extract in an alcohol (e.g. methyl alcohol) and elution on a basic ion-exchange resin, e.g. Amberlite IRA 400® or Relite 2A®.

The eluted neutral fraction is then subjected to treatment with concentrated alkalin and extraction with a water immiscible solvent.

The alcohols mixture of the present invention, which is obtained with a yield of about 0.3-0.5% by weight with respect to the starting vegetal material, according to different chromatographic techniques and careful spectroscopic analysis (MS, ¹H-NMR, ¹³C-NMR), is composed by three classes of compounds:

a) saturated and unsaturated long-chain alcohols containing a primary alcoholic moiety, totalling 20% of the mixture weight, having the relative composition reported hereinafter,

b) sterol and triterpenic alcohols, amounting to 40% of the mixture, having the relative composition reported hereinafter:

c) unsaturated alcohols having the polyprenolic structure (I), amounting to 40% of the mixture weight, having the relative composition reported hereinafter.

The quantitative compositions of a-b-c may sometimes show variations according to the vegetal material used.

The antiandrogenic activity of the fraction obtained according to the invention (FA), has been tested in the orchiectomized rat treated with exogenous androgens. In particular, mole rats were orchiectomized and subcutaneously treated for 6 days with testosterone propionate dissolved in olive oil at the dose of 12 mg die. Contemporaneously to the steroid treatment, the animals were treated with doses of 50 mg die of the above alcoholic fraction or 400 mg die of total lipophilic extract of S. repens (EL) as reference.

At the end of the experiment, the ventral prostata and the seminal vesicles of the treated animals and of the controls were weighed.

Both the tested products decreased in a statistically significant way the weight of these organs, as shown in Table 1. However, the alcoholic fraction, administered at a dose 8 time lower than that of the total extract, has surprisingly induced a larger percent decrease of the organs weight.

Table 1 - Antiandrogenic effect of the total lipophilic extract (EL) of S. repens and of the alcoholic fraction (FA) contained therein on the weight of the ventral prostata and of the seminal vesicles of orchiectomized rats treated with 12 mg die of testosterone palmitate (TP).

| Rats | N. animals | Body weight | Ventral prostata mg | Ventral prostata mg/100 g body weight | Seminal vesicles mg | Seminal vesicles mg/100 g body weight |
|---|---|---|---|---|---|---|
| Normal controls | 15 | 113±3 | 36.9±2.7 | 32.9±2.6 | 33.1±2.9 | 29.7±2.9 |
| Orchiectomized controls | 15 | 107±4 | 5.1±1.2 | 4.9±1.3 | 15.2±1.1 | 14.1±0.7 |
| Orchiectomized + TP | 15 | 112±2 | 30.2±3.0 | 27.4±2.7 | 26.1±1.7 | 23.3±1.4 |
| Orchiectomized + TP + FA (50 mg) | 15 | 104±3 | 14.3±1.3 (53) ° | 13.7±1.4 (50) ° | 18.6±1.5 (29) ° | 17.8±0.2 (24) ° |
| Orchiectomized + TP + EL (400 mg) | 15 | 108±4 | 18.7±1.8 (38) ° | 17.3±1.6 (37) ° | 22.7±1.6 (13) ° | 21.0±1.0 (10) ° |

° Percent decrease of the organs in respect to rats treated with TP only.

The absence of toxicity and the high antiandrogenic activity in the animal make the application of the extract containing the higher alcohols of the invention particularly advantageous in the therapy of benign prostatic adenoma.

For this aim, the extract of the invention is conveniently formulated in pharmaceutical compositions which may be administered by oral or rectal route, using conventional methods and excipients, such as those described in "Remington's Pharmaceutical Sciences Handbook", Hack Pub. Co., N.Y., U.S.A. The daily posology will depend of course on the patient's conditions (weight, sex, age) and on the seriousness of the disease: it will however generally range from 20 to 100 mg, divided in two or more administrations per day.

The following examples illustrate the invention without limiting it.

**EXAMPLE 1**

Finely ground fruits and seeds of S. repens (1 kg) were extracted at room temperature with four 1.3 l portions of n-hexane, each extraction being carried out for 12 hours.

The percolates were concentrated under vacuum up to total elimination of the solvent and the residue (90 g) was treated for 48 hours at room temperature with 4 l of methanol and 1.2 l of 2.5 N KOH. The reaction mixture was filtered from the separated solid and the mother liquors were diluted with 15 l of purified water

Two extractions with two 3 l portions of methylene chloride were carried out and the organic phases were washed with 2 l of a solution made acid by IN hydrochloric acid, then with water and finally dried ($Na_2SO_4$). After evaporation, 3 g of a pole yellow oily residue were obtained, comprising a mixture of alcohols.

The mixture of the compositions has been evaluated by the following methods:

1 g of product, subjected to column chromatography containing 100 g of silica gel, gave, after elution with methylene chloride and increasing amounts of ethyl acetate, three fractions:

- **Fraction 1 (200 mg).**

At the GLC-MS exam, it shows after silylation the following composition:

| | relative abundance | $M^+$ |
|---|---|---|
| farnesol | 0.18 | 294 |
| phytol | 8.83 | 368 |
| geranylgeraniol | 6.88 | 362 |
| $n-C_{22}H_{45}OH$ | 0.18 | 383 (M-15) |
| $n-C_{23}H_{47}OH$ | 0.33 | 411 (M-15) |
| $n-C_{24}H_{49}OH$ | 1.00 | 425 (M-15) |
| $n-C_{26}H_{53}OH$ | 1.44 | 439 (M-15) |
| $n-C_{28}H_{57}OH$ | 2.20 | 467 (M-15) |

whose retention times and mass spectra have been compared with authentic samples.

- **Fraction 2 (400 mg).**

After treatment with 3 ml of acetic anhydride in 2 ml of pyridine and fractioning on column containing 80 g of silica gel impregnated with 10% silver nitrate (eluent petroleum ether and increasing amounts of methylene chloride), the following sterolic and triterpenic alcohols in form of acetate have been isolated from this fraction:

| | relative abundance | $M^+$ | p.f. °C |
|---|---|---|---|
| stigmasterol acetate | 1.62 | 394 (M-AcOH) | 142° |
| ß-sitosterol | 12.23 | 396 (M-AcOH) | 126-7° |
| cycloartenol | 3,97 | 468 | 123° |
| lupeol | 2.40 | 468 | 220° |
| 24-methylenecycloartanol | 1.00 | 482 | 104-5° |

The chemico-physical and spectroscopic features (IR, $^1$H-NMR, MS) of the isolated products turn out to be identical to those of authentic sample.

- **Fraction 3 (400 mg).**

This fraction, composed by polyprenals, has been fractioned by column chromatography containing 90 g of Lichroprep RP 18® (Merck) with 90% aqueous acetone.

The following polyprenols having general structure (I) have been isolated:

| | relative abundance | M+ (as TMS-derivative) |
|---|---|---|
| n = 1 | 1.00 | 498 |
| n = 2 | 7.00 | 566 |
| n = 3 | 54.00 | 634 |
| n = 4 | 3.80 | 702 |

whose configuration has been determined by means of proton and carbon nuclear magnetic resonance. In particular, the $^{13}$C-NMR pectra of the polyprenols show the following methyl signals:

17.7 (terminal trans Me), 25.7 (terminal cis Me), 23.4 (3 cismethyl signal), 16.0 (n trans methyl signals), 23.4 ppm (Me in C-3).

Qualitatively and quantitatively identical results were obtained by carrying out the extraction with hypercrytical $CO_2$ and subjecting the extract to the above described treatments.

## EXAMPLE 2

The residue (90 g) obtained from the hexane extraction according to Example 1 of 1 kg of fruits of Serenoa repens, was taken up with 2.2 l of methanol. The obtained solution was filtered from the undissolved particles and eluted on a column containing 3 l of strong basic resin Amberlite IRA 400®.

The neutral methanolic eluate, containing 13.5 g of residue, was concentrated under vacuum up to a volume of 300 ml and treated at room temperature for 24 hours with 100 ml of 2.5 N KOH aqueous solution. The reaction mixture was filtered from the separated solid and the mother liquors, diluted with 1.5 l of water, were extracted with 2 × 200 ml di methylene chloride. The organic phases were collected, washed to neutrality with water and dried ($Na_2SO_4$). After evaporation under vacuum 3 g of oily residue were obtained, composed by a mixture of alcohols identical to that described in Example 1.

## EXAMPLE 3

**Tablet pharmaceutical composition containing the preparation consisting of natural alcohols.**

Each 100 mg tablet contains:
Alcoholic fraction extracted from S. repens mg 20
Granular cellulose mg 40
Crospovidone® mg 20
Sodium carboxymetylcellulose mg 10
Polyvinylpyrrolidone PM 30.000 mg 3
Colloidal silica mg 5
Talc mg 2

**EXAMPLE 4**

**Suppository pharmaceutical formulation containing the preparation consisting of natural alcohols**

Each 1.5 g suppository contains:
Alcoholic fraction extracted from S. repens mg 50
Semisynthetic glycerides F.U. (excipient for suppositories) mg 1450

**Claims**

Claim for the following Contracting States : ES, GR

1. A process for the preparation of Serenoa repens extracts consisting of long chain saturated and unsaturated, sterolic and triterpenic alcohols characterized in that the total lipophilic extract, optionally preventively purified from the acidic component by elution on ion-exchange resins, is subjected to alkaline hydrolysis in hydroalcoholic solvents followed by optional filtration and extraction of the filtrate with a water immiscible solvent.

European Patent Office

# EUROPEAN SEARCH REPORT

Application Number

EP 88 10 5681

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS/RRM, vol. 33, 1987, ref. 125151, Philadelphia, PA, US; A. MANDRESSI et al.: "Treatment of uncomplicated benign prostatic hypertrophy BPH by an extract of serenoa-repens clinical results" & J ENDROCRINOL INVEST 1987. VOL 10, NO SUPPL. 2 P49<br>* Abstract *<br>--- | 1 | A 61 K 35/78<br>A 61 K 31/045 |
| X,P | BIOLOGICAL ABSTRACTS, vol. 85, 1988, ref. 20030, Philadelphia, PA, US; J.OLLE CARRERAS: "Our experience with hexanic extract of serenoa-repens in the treatment of benign prostatic hypertrophy" & ARCHIVOS ESPANOLES DE UROLOGIA(SPAIN) 1987, 40(5), 310-313<br>* Abstract *<br>--- | 1 | |
| Y,P | BE-A-1 000 036 (LAMPUGNANI FARMACEUTICI S.P.A.)<br>* Page 3, lines 7-17; page 7, lines 4-13 *<br>--- | 1 | **TECHNICAL FIELDS SEARCHED (Int. Cl.4)** |
| Y | EP-A-0 068 055 (PIERRE FABRE S.A.)<br>* Claims *<br>--- | 1 | A 61 K |
| Y | FR-A-2 480 754 (PIERRE FABRE S.A.)<br>* Claims *<br>--- | 1 | |
| Y | BIOLOGICAL ABSTRACTS, vol. 78, 1984, ref. 94399, Philadelphia, PA, US; J.P. TARAYRE et al.: "Anti.edematous action of an hexane extract from Serenoa repens drupes" & ANN PHARM FR 41(6): 559-570. 1983[recd. 1984]<br>* Abstract *<br>--- -/- | 1 | |

The present search report has been drawn up for all claims

| Place of search | Date of completion of the search | Examiner |
|---|---|---|
| THE HAGUE | 25-07-1988 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 10 5681

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 99, no. 2, 11th July 1983, page 296, abstract no. 10736c, Columbus, Ohio, US; P. HATINGUAIS et al.: "Composition of the hexane extract from Serenoa repens Bartram fruits" & TRAV. SOC. PHARM. MONTPELLIER 1981, 41(4), 253-62 * Abstract * --- | 1 | |
| A | FR-A-2 556 968 (P.F. MEDICAMENT) ----- | | |

TECHNICAL FIELDS SEARCHED (Int. Cl.4)

The present search report has been drawn up for all claims

| Place of search | Date of completion of the search | Examiner |
|---|---|---|
| THE HAGUE | 25-07-1988 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document